**Europäisches Patentamt**

(19) **European Patent Office**

**Office européen des brevets**

(11) **EP 1 427 381 B1**

(12) **EUROPÄISCHE PATENTSCHRIFT**

(45) Veröffentlichungstag und Bekanntmachung des Hinweises auf die Patenterteilung:
**19.10.2005  Patentblatt 2005/42**

(51) Int Cl.$^7$: **A61K 7/40**

(21) Anmeldenummer: **02738011.2**

(86) Internationale Anmeldenummer:
**PCT/EP2002/004751**

(22) Anmeldetag: **30.04.2002**

(87) Internationale Veröffentlichungsnummer:
**WO 2002/087518 (07.11.2002 Gazette 2002/45)**

(54) **HAUTSCHUTZPRODUKTE AUF DER BASIS MULTIPLER EMULSIONEN**

USE OF MULTIPLE EMULSIONS AS SKIN PROTECTION PRODUCTS

UTILISATION D'EMULSIONS MULTIPLES COMME PRODUITS DE PROTECTION DE LA PEAU

(84) Benannte Vertragsstaaten:
**AT BE CH CY DE DK ES FI FR GB GR IE IT LI LU MC NL PT SE TR**

(30) Priorität: **30.04.2001  DE 10120927**

(43) Veröffentlichungstag der Anmeldung:
**16.06.2004  Patentblatt 2004/25**

(73) Patentinhaber: **Stockhausen GmbH**
**47805 Krefeld (DE)**

(72) Erfinder:
 • **VEEGER, Marcel**
  **47874 Goch (DE)**
 • **KLOTZ, Andreas**
  **41516 Grevenbroich (DE)**
 • **BLÄSER, Edeltraud**
  **47803 Krefeld (DE)**
 • **SEIDL, Silke**
  **47877 Willich (DE)**

(56) Entgegenhaltungen:
 **DE-A- 19 612 084       US-A- 4 931 210**
 **US-B1- 6 174 518**

 • **5. Jahrestagung der Gesellschaft für Dermopharmazie, Universität Zürich, Abstract des Vortrags von Herrn A. Klotz am 28.03.2001, verfügbar unter http://www.gd-online.de/ XP002217808**
 • **KAWA, R; ANSMANN, A; JACKWERTH, B; LEONARD, M: "Das Synergistic-Sun-Systems-Konzept" PARFÜMERIE UND KOSMETIK, Bd. 80, Nr. 3, 1999, Seiten 17-23, XP001117732**

**Beschreibung**

Die Erfindung betrifft die Verwendung von multiplen Emulsionen als Hautschutzprodukte zum Schutz gegen wässrige Noxen sowie die entsprechenden Produkte.

Hautschutzprodukte sind Schutzmittel gegen Schädigungen der Haut, die z.B. durch Witterungseinflüsse, Wasser und wässrige Lösungen, Chemikalien und vor allem industrielle Verschmutzungen, wie gefährliche oder stark schmutzende Arbeitsstoffe, hervorgerufen werden können. Solche Hautschutzprodukte, die insbesondere das Einwirken von Arbeitsstoffen auf die Haut verhindern sollen, überziehen die Haut mit einem sogenannten Barrierefilm, der dann eine Schutzbarriere gegen reizende und schädigende Stoffe ist.

Übliche Barrieremittel sind vor allem Paraffin-Kohlenwasserstoffe wie mineralische Öle, Vaseline etc. aber auch mineralische und pflanzliche Wachse einschließlich Siliconöle und Silikonwachse. Im Handel werden Hautschutzpräparate in den unterschiedlichsten Zubereitungsformen dargeboten, wobei Hautsalben, Hauteremes, Hautlotionen, Hautöle und Hautgele die wichtigsten darstellen. Hautcremes und Hautlotionen basieren vor allem auf Emulsionen vom O/W- (Öl in Wasser) oder W/O- Typ (Wasser in Öl). Hauptbestandteile der Öl-Phase (auch Fett- oder Lipidphase) können dann Fettalkohole, Fettsäuren, Fettsäureester, Wachse, Vaseline, Paraffine aber auch sonstige Fette und Öle hauptsächlich natürlichen Ursprungs sein.
Die wässrige Phase kann u.a. wasserlösliche Pflegewirkstoffe, die feuchtigkeitsregulierend bzw. feuchtigkeitsbewahrend sind, enthalten.

Welche der vorgenannten Emulsionstypen in einem Hautschutzprodukt Anwendung finden, richtet sich vornehmlich danach, welcher Schutzzweck mit dem Produkt verfolgt werden soll bzw. gegen welche Arbeitsstoffe das Produkt schätzen soll.
Neben den für Hautschutzprodukte üblicherweise verwendeten Emulsionen vom O/W- oder W/O- Typ werden auch multiple Emulsionen zur Herstellung von kosmetischen und pharmazeutischen Produkten beschrieben.

Bei solchen multiplen Emulsionen handelt es sich um Emulsionen von Emulsionen, deren wichtigste Vertreter multiple Wasser/Öl/Wasser (W/O/W)- sowie Öl/Wasser/Öl (O/W/O)- Emulsionen vielfach in der Patentliteratur beschrieben sind.

So werden in der DE 41 31 678 A1 multiple Emulsionen beschrieben, die in kosmetischen Hautpflegeprodukten aber auch in medizinischen topischen Zubereitungen Anwendung finden können.
Wasser und wässrige Lösungen mäßig hautirritierender Substanzen können bei wiederholtern Hautkontakt über einen längeren Zeitraum ein kumulativ toxisches Kontaktekzern (Abnutzungsdermatose) verursachen. Zum Schutz der Haut vor solchen Belastungen werden üblicherweise wasserunlösliche Barrierepräparate angewendet die einen Schutzfilm auf der Haut bilden. Nachteilig an solchen Präparaten ist jedoch, daß die natürliche Wasserdampfabgabe über die Haut beeinträchtigt wird. Aufgrund des damit verbundenen Wärme- und Feuchtigkeitsstans wird die Akzeptanz für die Anwendung solcher Produkte durch Beschäftigte, die täglich vielfach mit wässrigen Hautschadstoffen in Berührung kommen, herabgesetzt.
Eine bessere Akzeptanz weisen hydrophile Formulierungen vom Typ O/W (Öl in Wasser) auf, die ihre Schutzwirkung über hohe Anteile enthaltener Silikonverbindungen erhalten.

Silikone, wie z.B. Silikonöle und Silikonwachse sind hervorragende Barrieremittel, die in Hautschutzmitteln eine besondere Stabilität aufweisen. So sind sie wärmebeständig und gegen die Einwirkung von ätzenden Chemikalien außerordentlich beständig. Darüber hinaus sind solche Silikonpräparate stark hydrophob und hautungefährlich, weil sie physiologisch verträglich, d.h. nicht gesundheitsschädlich, sondern auch hautverträglich sind. Aufgrund der geringen Oberflächenspannung von Silikonölen lassen sie sich leicht auf der Haut verteilen. Vorteilhaft ist auch, daß bei Silikonschichten auf der Haut im Gegensatz zu Paraffinen, Vaseline etc. keine Gefahr von Wärmestanungen auf der Haut besteht.

So beschreibt auch die DE 4131678 A1 eine Hautschntzemulsion, die als Silikonverbindung Cyclomethicone enthält, wobei die W/O/W-Emulsion durch den Einsatz von ethoxylierten Fettalkoholen als Emulgatoren zustande kommt.

Nachteilig an solchen silikonhaltigen Präparaten ist jedoch, daß diese Präparate auf Gegenständen z.B. auf Werkstoffen bzw. Werkstücken Rückstände hinterlassen können, wenn solche Werkstücke per Hand einem weiteren Arbeitsgang zugeführt werden. So können z.B. die Beschäftigten bei zu lackierenden Werkstücken solche silikonhaltigen Hautschutzmittel nicht einsetzen, da diese sehr schwer entfernbaren Silikonrückstände bei der Weiterverarbeitung dieser Werkstücke wie z. B. Lackieren oder Vulkanisieren stark störend wirken. So können, insbesondere in der Automobil-, Lack- und gummiverarbeitenden Industrie silikonhaltige Hautschutzprodukte trotz ihrer hervorragenden Schutzwirkung und Akzeptanz nicht zum Einsatz kommen.

Es besteht somit ein großes Bedürfnis nach silikonölfreien Hautschutzmitteln, die eine vergleichbar gute Wirksamkeit beim Schutz gegenüber Wasser und wässrigen, hautirritierenden Lösungen aufweisen.

Aufgabe der vorliegenden Erfindung war es daher, solche silikonfreien Hautschutzmittel zum Schutz gegen wässrige Noxen zu entwickeln, die darüber hinaus eine hohe kosmetische Akzeptanz, wie z.B. Hautpflegeprodukte haben.

Überraschenderweise wurde gefunden, daß solche Hautschutzmittel erhalten werden können, die unter Verwendung von multiplen W/O/W-Emulsionen hergestellt worden sind, wobei die W/O/W-Emulsionen unter Verwendung einer Emulgatormischung aus Polyolpoly-12-hydroxystearaten in Kombination mit einem Alkyl- und/oder Alkylenglucosid und einem Fettalkohol und/oder Partialgleriden gebildet werden und die Emulgatormischung zusätzlich mindestens ein ethoxyliertes Dipolyhydroxystearat enthält und der Anteil der unpolaren Öle 20 Gew.-% im Hautschutzmittel nicht übersteigt.

Die Herstellung solcher multiplen Emulsionen bzw. W/O/W-Emulsionen, die unter dem Aspekt der Hautpflege entwickelt worden sind, werden in der DE 196 12 084 A1 beschrieben, auf die vollinhaltlich Bezug genommen und hiermit in die Beschreibung dieser Patentanmeldung mitaufgenommen wird.

Erfindungsgemäß wird für ein Hautschutzmittel die Emulgatormischung in Mengen von 1 bis 25 Gew.-%, vorzugsweise 5 bis 15 Gew.-% - bezogen auf die Gesamtzusammensetzung des Hautschutzmittels - eingesetzt.

Hierbei enthält die Emulgatormischung mindestens ein Polyolpoly-12-hydroxystearat als Emulgatorkomponente, vorzugsweise in einer Menge von 1 bis 5 Gew.-% - bezogen auf die Gesamtzusammensetzung des Hautschutzmittels, wobei Polyglycerinpoly-12-hydroxystearate, die unter der Marke Dehymuls® PGPH von der Firma Henkel KGaA, Düsseldorf im Handel erhältlich sind, als Emulgatorkomponente besonders bevorzugt sind.

Vorteilhaft können der Emulgatormischung weitere Coemulgatoren zugefügt werden. Vorzugsweise kann der Emulgatormischung zusätzlich mindestens ein ethoxyliertes Dipolyhydroxystearat, insbesondere in einer Einsatzmenge von 0,1 bis 1,0 Gew.-% - wiederum bezogen auf die Gesamtzusammensetzung des Hautschutzmittels - zugemischt werden, wobei als Coamulgatoren PEG - 30 Dipolyhydroxystearate besonders bevorzugt sind, die unter der Bezeichnung ARLACEL P135 im Handel erhältlich sind. Weitere einsetzbare Coemulgatoren sind dem Fachmann bekannte hydrophile Emulgatoren, die ebenfalls in einer Einsatzmenge von 0,1 bis 1,0 Gew.-% - bezogen auf die Gesamtmenge des Mittels - der Emulgatormischung zugesetzt werden können. Als Beispiel sei hier PEG-400-Stearat genannt.

Die Entwicklung der multiplen W/O/W-Emulsion zeigte, daß bei einer sehr guten Wirksamkeit gegenüber wässrigen Noxen eine sehr gute kosmetische Akzeptanz (vergleichbar einer leichten Pflegecreme) vorliegen kann, wenn der Anteil der unpolaren. Öle 20 Gew.-%, vorzugsweise 15 Gew.-% und besonders bevorzugt 10 Gew.-% nicht übersteigt.

Als unpolare Öle werden die üblicherweise in kosmetischen bzw. pharmazeutischen Zubereitungen einsetzbaren Öle verwendet, die die Haut mit einem Schutz- oder Barrierefilm überziehen, insbesondere die bereits oben genannten Paraffin-Kohlenwasserstoffe wie mineralische Öle, beispielsweise Vaseline etc. einschließlich mineralischer und pflanzlicher Wachse, soweit diese nicht wie z.B. die silikonhaltigen Präparate zu unerwünschten Rückständen auf Werkstoffen und Werkstücken führen.

Aufgrund der Polarität dieser Öle wird eine hervorragende Schutzwirkung gegenüber hydrophilen Hautschadstoffen erreicht. Ergänzend können diese unpolaren Öle noch Zusätze wie z.B. Isopropylpalmitat oder Isopropylmyristat aufweisen oder andere dem Fachmann bekannte die streichfähigkeits- oder die festigkeitserhöhende Additive aufweisen.

Darüber hinaus wurde gefunden, daß durch den Zusatz von 0,1 bis 5 Gew.-%, vorzugsweise 0,5 bis 2 Gew.-% und besonders bevorzugt 0,5 bis 1,5 Gew.-% Bisabolol eine Verbesserung der Barriere, insbesondere auch bei bereits vorgeschädigter Haut erreicht werden kann, wodurch eine wirksame Unterstützung der Hautregeneration bewirkt wird. Vorteilhaft ist weiterhin die Zugabe von natürlichen pflanzlichen Gerbstoffen, vorzugsweise in einer Menge von 0,1 bis 5 Gew.-%, bezogen auf die Gesamtmenge des Hautschutzmittels, wobei Hamamelis virginiana als Gerbstoff besonders bevorzugt ist.

Durch die erfindungsgemäße Kombination von Emulsionsgrundlage, unpolarer Öle und insbesondere Bisabolol konnten die positiven Effekte des Hautschutzes, der Hautregeneration und der kosmetischen Akzeptanz in einer Formulierung vereinigt werden. Dies war insofern überraschend, weil die unpolaren Öle, die normalerweise einen eher hinderlichen Effekt zur Hautregeneration und kosmetischen Akzeptanz mit sich bringen, jedoch für den Hautschutz ein wertvoller Bestandteil sind, in der multiplen W/O/W-Emulsion positive Effekte im Sinne des Hautschutzes, der Hautregeneration und der kosmetischen Akzeptanz bewirken.

Insbesondere zeigen die erfindungsgemäß unter Verwendung von multiplen Emulsionen hergestellten Hautschutzmittel einen hervorragenden Schutz vor wässrigen Noxen, so daß guter Hautschutz auch bei Feuchtarbeiten gemäß TRGS 531 gewährleistet ist. Somit können solche Hautschutzmittel vorteilhaft für Arbeitsplätze mit wässrigen Hautbelastungen, z.B. in der lebensmittelverarbeitenden, metallverarbeitende, gummiverarbeitende Industrie. Krankenhäusern, Land- und Forstwirtschaft aber auch bei entsprechenden Freizeit-, Hobby- und Haushaltsaktivitäten z.B. Garten- und Spülarbeiten Anwendung finden.

Darüber hinaus gibt es keine Beeinträchtigung von Arbeitsprozessen durch Rückstände von Silikonverbindungen z. B. auf Werkstoffen bzw. Werkstücken.

Die nachfolgenden Beispiele sollen die vorliegende Erfindung verdeutlichen. Die Zahlenwerte in den Beispielen bedeuten Gewichtsprozente, bezogen auf das Gesamtgewicht der jeweiligen Zubereitungen.

| Beispiele bevorzugter Hautschutzformulierungen: (alle Angaben in Gewichts-%) | | | |
|---|---|---|---|
| | A | B | C |
| Mischung von C16/18-Alkylglykoside und Fettalkohol | 3,00 | 4,00 | 5,00 |
| Polyglycerinpoly-12-hydroxystearat | 2,00 | 3,00 | 3,50 |
| PEG-30 Dipolyhydroxystearate | 0,50 | 0,50 | 1,00 |
| PEG 40 Stearat | 0,50 | | |
| Paraffinöl | 6,00 | 8,00 | 10,00 |
| Vaseline weiß DAB | 1,00 | 2,00 | 3,00 |
| Isopropylpalmitat | 3,00 | 3,00 | 3,00 |
| C12/15- Alkylbenzoate | 1,50 | 2,00 | 3,00 |
| Cetylstearylalkohol | 1,00 | 1,00 | 1,00 |
| Stearinsäure | 1,50 | 3,00 | 4,00 |
| Bisabolol | 0,50 | 1,00 | 2,00 |
| 1,2-Propandiol | 1,00 | 2,00 | 3,00 |
| Konservierungsmittel | q.s. | q.s. | q.s. |
| pflanzlicher Gerbstoff | 0,50 | 1,00 | 2,00 |
| Wasser | -ad 100- | | |

**Testmethoden:**

1.) Hautschutzwirkung

Die Hautschutzwirkung wurde im folgenden "in vivo"-Testmodell zur Überprüfung der Schutzwirkung von Hautschutzprodukten gegenüber dem ModellschadstoffNatriumlaurylsulfat (0,5 %ig in Wasser - im nachfolgenden als NaLS bezeichnet) als Referenzsubstanz für wässrige Hautschadstoffe überprüft.

Hierbei werden die Hautschutzpräparate über 1 Woche zweimal täglich auf die Haut aufgetragen und hierauf anschließend die Modellschadstoffe appliziert. Jeweils täglich wird die Hautreaktion visuell beurteilt und wichtige Parameter mit nicht hautbeeinflussenden Meßmethoden (z.B. TEWL) ermittelt. Pro Untersuchungsreihe können bis zu 7 Prüfprodukte getestet werden.

Das Testkollektiv besteht aus mindestens 5 hautgesunden Männern und Frauen im Alter von 18 bis 60 Jahren.

**Tag 1 bis 4:**

Von den auf dem volaren Unterarm gekennzeichneten Testflächen wird der Hautzustand (instrumentell mittels TEWL) ermittelt. Vor der Behandlung wird der TEWL0 ermittelt. 0,1 g Hautschutzprodukt werden unter Einhaltung einer Randomisierung auf 3 cm x 3 cm Fläche (9 cm$^2$) der volaren Seite des Unterarms appliziert und mit einem Gummifingerling oder einem Latexhandschuh in die Haut eingerieben. Ein Feld ohne Hautschutzprodukt mit Modellirritants dient als Negativkontrolle.

10 Minuten nach Produktauftrag wird der Überschuss mit einem Zellstofftuch abgetupft. Danach erfolgt die Applikation von Finn-Kammern mit Schadstoff auf alle Testfelder für 30 Minuten (12 mm Kammer: 50 µl; 18 mm Kammer 200 µl).Danach erfolgt die Abnahme der Finn-Kammern und das Abspülen des Armes mit Leitungswasser. 3h ±0,5h nach der ersten Applikation erfolgt eine Wiederholung der Hautschutz- und Schadstoffapplikation

An Tag 5 erfolgt eine abschließende Beurteilung der Hautreaktion ohne weitere Produktapplikation (TEWL5)

Die Auswertung erfolgt anhand der nachfolgenden Formel:

$$(\text{gemittelt TEWL5}_{\text{mit Hautschutz}} - \text{gemittelt TEWL0}_{\text{mit Hautschutz}}) /$$

$$(\text{gemitteltTEWL5}_{\text{ohne Hautschutz}} - \text{gemitteltTEWL0}_{\text{ohne Hautschutz}}) \times 100\%$$

Der Kontrollwert (mit 0,5% NaLS ohne Hautschutz /// gemittelt TEWL5$_{\text{ohne Hautschutz}}$ - gemitteltTEWL0$_{\text{ohne Hautschutz}}$) wird mit einer 100%igen Barriereschädigung gleichgesetzt. Daraus ergibt sich für die unterschiedlichen Hautschutzprodukte folgendes Ergebnis, das in Diagramm I graphisch dargestellt ist:

Die prozentuale Barriereschädigung gemessen am TEWL im repetetiven okklusiven Irritationstest (entsprechend Contact Dermatitis 2000, 42, 336 bis 343) konnten durch den zusätzlichen Einsatz der oben beschriebenen W/O/W-Formulierungen auf 48% gesenkt werden. Durch den Einsatz einer handelsüblichen W/O-Hautschutzformulierung konnte lediglich eine Reduktion der Hautveränderungen auf 71% erzielt werden. Durch die Vaseline DAB, die eine sehr schlechte Akzeptanz hat, konnten die Hautveränderungen auf 64 % gesenkt werden.

Demnach ist es möglich mit den erfindungsgemäßen W/O/W-Formulierungen Hautschutzprodukte herzustellen, die eine deutliche Wirkung gegenüber wässrigen Noxen besitzen. Dies war insofern überraschend, da handelsübliche O/W-Formulierungen, deren äußere Phase aus Wasser besteht, keine Wirkung gegenüber wässrigen Noxen zeigen.

2.) Hautpflegewirkung

Darüberhinaus weisen die erfindungsgemäßen silikonfreien Hautschutzmittel, die unter Verwendung der hier beschriebenen multiplen W/O/W-Emulsionen hergestellt wurden, auch regenerative Pflegeeffekte auf, wie anhand der nachfolgend beschriebenen Testmethode gezeigt wird:

Bei der verwendeten Methode handelt es sich ebenfalls um ein "in vivo"-Testmodell zur Überprüfung der Regenerationswirkung von Hautschutzprodukten. Die erforderliche Hautschädigung wird mit Natriumlaurylsulfat (0,5 %ig in Wasser) verursacht.

Hierbei werden die Hautschutzpräparate über 1 Woche zweimal täglich auf die vorher irritierte Haut aufgetragen. Jeweils täglich wird die Hautreaktion visuell beurteilt und wichtige Parameter mit nicht hautbeeinflussenden Meßmethoden (z.B. TEWL) ermittelt. Pro Untersuchungsreihe können bis zu 7 Prüfprodukte getestet werden.

Das Testkollektiv besteht aus mindestens 5 hautgesunden Männern und Frauen im Alter von 18 bis 60 Jahren.

**Tag 1 bis 4:**

Von den auf dem volaren Unterarm gekennzeichneten Testflächen wird der Hautzustand (instrumentell mittels TEWL) ermittelt. Vor der Behandlung erfolgt die Messung des TEWLO-Wertes.

Danach erfolgt die Applikation von Finn-Kammern mit Schadstoff auf alle Testfelder für 30 Minuten (12 mm Kammer: 50 $\mu$l; 18 mm Kammer 200 $\mu$l). Nach Abnahme der Finn-Kammern werden die Arme mit Leitungswasser abgespült und trockengetupft.

Anschließend werden 0,1 g Hautschutzprodukt unter Einhaltung einer Randomisierung auf 3 cm x 3 cm Fläche (9 cm$^2$) der volaren Seite des Unterarms appliziert und mit einem Gummifingerling oder einem Latexhandschuh in die Haut eingerieben. Ein Feld ohne Hautschutzprodukt mit Modellirritants dient als Negativkontrolle. 10 Minuten nach Produktauftrag wird der Überschuss mit einem Zellstofftuch abgetupft. 3h $\pm$0,5h nach der ersten Applikation erfolgt eine Wiederholung der Schadstoff- und Produktapplikation

An Tag 5 erfolgt eine abschließende Beurteilung der Hautreaktion ohne weitere Produktapplikation (TEWL5).

**Auswertung:**

Für den Barriereschaden wurde oben bereits folgende Formel entwickelt:

$$\text{(gemittelt TEWL5}_{\text{mit Hautschutz}} - \text{gemittelt TEWL0}_{\text{mit Hautschutz}}) \; / $$

$$\text{(gemitteltTEWL5}_{\text{ohne Hautschutz}} - \text{gemitteltTEWL0}_{\text{ohne Hautschutz}}) \; \text{x } 100\%$$

Der Kontrollwert 0,5% NaLS ohne Hautschutz (gemitteltTEWL5$_{\text{ohne Hautschutz}}$ - gemitteltTEWL0$_{\text{ohne Hautschutz}}$) wird mit einer 100%igen Barriereschädigung gleichgesetzt. Nach dem Regenerationstestmodell wird der prozentuale Regenerationseffekt R wie folgt dargestellt:

$$R = 100\% - \text{prozentuale Barriereschädigung d.h.}$$

$$R = 100\% - \text{(gemittelt TEWL5}_{\text{mit Hautschutz}} - \text{gemittelt TEWL0}_{\text{mit Hautschutz}}) \; / $$

$$\text{(gemitteltTEWL5}_{\text{ohne Hautschutz}} - \text{gemitteltTEWL0}_{\text{ohne Hautschutz}}) \; \text{x } 100\%$$

Daraus ergibt sich für die unterschiedlichen Hautschutzprodukte folgendes Ergebnis, das auch in Diagramm II graphisch dargestellt ist:

Die prozentuale Barriereschädigung gemessen am TEWL im Regenerationsmodell konnten durch den Einsatz der .oben beschriebenen W/O/W-Formulierungen 92% gesenkt werden. Dies entspricht einem Regenerationseffekt von 8%. Durch den Einsatz einer handelsüblichen W/O-Hautschutzformulierung bzw. der Vaseline wurde der Barriereschaden in dem Modell mit 106,2% bzw. 145% sogar noch verstärkt. Dies ergibt einen negativen Regenerationseffekt bzw. eine Barriereschädigungsverstärkung um 6,2% bzw.45%.

Die ermittelten Ergebnisse zeigen, daß die erfindungsgemäßen Hautschutzformulierungen, die die oben beschriebenen. W/O/W Formulierungen enthalten, einen signifikanten regenerativen (Pflege)-Effekt aufweisen.

Demnach zeichnen sich die o.g. W/O/W-Formulierungen durch einen Hautschutzwirkung gegenüber der verwendeten wässrigen Modellnoxe NaLS und einem regenerativen Effekt bei einer Barriereschädigung aus.

Die handelsübliche W/O-Schutzformulierung, die in den Tests gegenüber wässrige Noxen eingesetzt wurde und den derzeitigen Stand der Technik darstellt, ist wie folgt nach INCI zusammengesetzt:

Wasser, Paraffinum Liquidum, Petrolatum, Talc, Zinc Oxide, Glycerin, Methyl Glucose Dioleate, Caprylic/Capric Triglyceride, Isohexadecan, PEG-45/Dodecyl Glycol Copolymer, C18-36 Acid Triglyceride, Magnesium stearate, C12-15 AlkylBenzoate, Magnesium Sulfat, Parfüm, Sodium Bischlorophenylsulfamine, Phenoxyethanol, Methyl Parabene, Ethylparabene, Propylparabene, Butylparabene, Isobutylparabene, Lactic Acid.

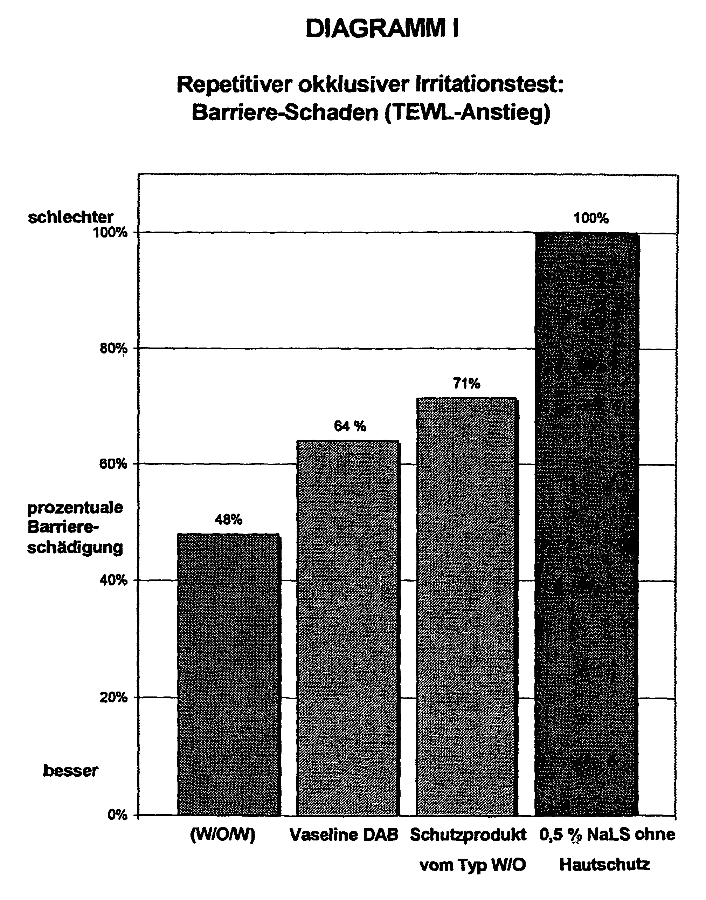

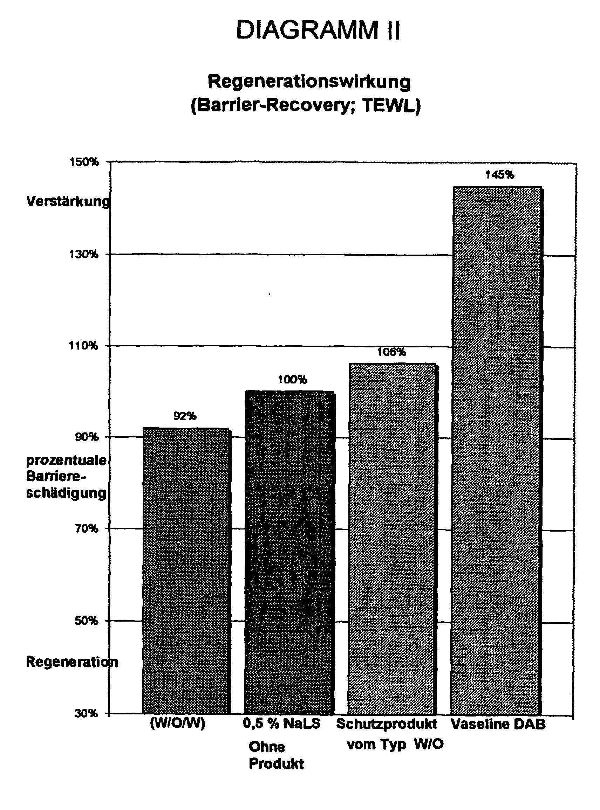

**Patentansprüche**

1. Verwendung von multiplen W/O/W-Emulsionen zur Herstellung von silikonfreien Hautschutzmitteln zum Schutz gegen wässrige Noxen, **dadurch gekennzeichnet, daß** die W/O/W-Emulsionen unter Verwendung einer Emulgatormischung aus Polyolpoly-12-hydroxystearaten in Kombination mit einem Alkyl- und/oder Alkylenglucosid und einem Fettalkohol und/oder Partialglyceriden gebildet wird, wobei die Emulgatormischung zusätzlich mindestens ein ethoxyliertes Dipolyhydroxystearat enthält und der Anteil der unpolaren Öle 20 Gew.-% im Hautschutzmittel nicht übersteigt.

2. Verwendung nach Anspruch 1, **dadurch gekennzeichnet, daß** man die Emulgatormischung in Mengen von 1 bis 25 Gew.-%, bezogen auf die Gesamtzusammensetzung des Hautschutzmittels, einsetzt.

3. Verwendung nach den Ansprüchen 1 bis 2, **dadurch gekennzeichnet, daß** man die Emulgatormischung in Mengen von 5 bis 15 Gew.-%, bezogen auf die Gesamtzusammensetzung des Hautschutzmittels, einsetzt.

4. Verwendung nach den Ansprüchen 1 bis 3, **dadurch gekennzeichnet, daß** die Emulgatormischung mindestens ein Polyolpoly-12-hydroxystearat als Emulgatorkomponente enthält.

5. Verwendung nach den Ansprüchen 1 bis 4, **dadurch gekennzeichnet, daß** die Emulgatormischung als Polyolpoly-12-hydroxystearate Polyglycerinpoly-12-hydroxystearate als Emulgatorkomponente enthält.

6. Verwendung nach den Ansprüchen 1 bis 5, **dadurch gekennzeichnet, daß** als ethoxylierte Dipolyhydroxystearate PEG-30 Dipolyhydroxystearate eingesetzt werden.

7. Verwendung nach den Ansprüchen 1 bis 6, **dadurch gekennzeichnet, daß** der Anteil der unpolaren Öle 15 Gew.-% im Hautschutzmittel nicht übersteigt.

8. Verwendung nach den Ansprüchen 1 bis 7, **dadurch gekennzeichnet, daß** der Anteil der unpolaren Öle 10 Gew.-% im Hautschutzmittel nicht übersteigt.

9. Verwendung nach den Ansprüchen 1 bis 8, **dadurch gekennzeichnet, daß** Bisabolol in einer Menge von 0,1 bis 5 Gew.-% im Hautschutzmittel enthalten ist.

10. Verwendung nach den Ansprüchen 1 bis 9, **dadurch gekennzeichnet, daß** Bisabolol in einer Menge von 0,5 bis 1,5 Gew.-% im Hautschutzmittel enthalten ist.

11. Verwendung nach den Ansprüchen 1 bis 10, **dadurch gekennzeichnet, daß** natürliche pflanzliche Gerbstoffe in einer Menge von 0,1 bis 2 Gew.-% im Hautschutzmittel enthalten sind.

12. Verwendung nach den Ansprüchen 1 bis 11, **dadurch gekennzeichnet, daß** natürliche pflanzliche Gerbstoffe in einer Menge von 0,4 bis 0,8 Gew.-% im Hautschutzmittel enthalten sind.

**13.** Silikonfreie Hautschutzmittel zum Schutz gegen wässrige Noxen, erhältlich aus W/O/W-Emulsionen, die unter Verwendung einer Emulgatormischung aus Polyolpoly-12-hydroxystearaten in Kombination mit einem Alkyl- und/oder Alkylenglucosid und einem Fettalkohol und/oder Partialglyceriden gebildet werden, wobei die Emulgatormischung zusätzlich mindestens ein ethoxyliertes Dipolyhydroxystearat enthält und der Anteil der unpolaren Öle 20 Gew.-% im Hautschutzmittel nicht übersteigt.

**14.** Hautschutzmittel nach Anspruch 13, **dadurch gekennzeichnet, daß** man die Emulgatormischung in Mengen von 1 bis 25 Gew.-%, bezogen auf die Gesamtzusammensetzung des Hautschutzmittels, einsetzt.

**15.** Hautschutzmittel nach den Ansprüchen 13 bis 14, **dadurch gekennzeichnet, daß** man die Emulgatormischung in Mengen von 5 bis 15 Gew.-%, bezogen auf die Gesamtzusammensetzung des Hautschutzmittels, einsetzt.

**16.** Hautschutzmittel nach den Ansprüchen 13 bis 15, **dadurch gekennzeichnet, daß** die Emulgatormischung mindestens ein Polyolpoly-12-hydroxystearat als Emulgatorkomponente enthält.

**17.** Hautschutzmittel nach den Ansprüchen 13 bis 16, **dadurch gekennzeichnet, daß** die Emulgatormischung als Polyolpoly-12-hydroxystearate Polyglycerinpoly-12-hydroxystearate als Emulgatorkomponente enthält.

**18.** Hautschutzmittel nach den Ansprüchen 13 bis 17, **dadurch gekennzeichnet, daß** als ethoxylierte Dipolyhydroxystearate PEG-30 Dipolyhydroxystearate eingesetzt werden.

**19.** Hautschutzmittel nach den Ansprüchen 13 bis 18, **dadurch gekennzeichnet, daß** der Anteil der unpolaren Öle 15 Gew.-% im Hautschutzmittel nicht übersteigt.

**20.** Hautschutzmittel nach den Ansprüchen 13 bis 19, **dadurch gekennzeichnet, daß** der Anteil der unpolaren Öle 10 Gew.-% im Hautschutzmittel nicht übersteigt.

**21.** Hautschutzmittel nach den Ansprüchen 13 bis 20, **dadurch gekennzeichnet, daß** Bisabolol in einer Menge von 0,1 bis 5 Gew.-% im Hautschutzmittel enthalten ist.

**22.** Hautschutzmittel nach den Ansprüchen 13 bis 21, **dadurch gekennzeichnet, daß** natürliche pflanzliche Gerbstoffe in einer Menge von 0,1 bis 2 Gew.-% im Hautschutzmittel enthalten sind.

**23.** Hautschutzmittel nach den Ansprüchen 13 bis 22, **dadurch gekennzeichnet, daß** natürliche pflanzliche Gerbstoffe in einer Menge von 0,4 bis 0,8 Gew.-% im Hantschutzmittel enthalten sind.

## Revendications

**1.** Utilisation d'émulsions multiples E/H/E pour fabriquer des agents de protection de la peau sans silicone destinés à la protection contre des NOx aqueux,
**caractérisée en ce que**
les émulsions E/H/E sont préparées avec un mélange émulsifiant constitué de poly-12-hydroxystéarates de polyol en combinaison avec un glucoside d'alkyle et/ou d'alkylène et d'un alcool gras et/ou de glycérides partiels, le mélange émulsifiant contenant en outre au moins un dipolyhydroxystéarate éthoxylé et la fraction d'huiles apolaires dans l'agent de protection de la peau ne dépassant pas 20 % en poids.

**2.** Utilisation selon la revendication 1,
**caractérisée en ce que**
le mélange émulsifiant est utilisé dans des quantités de 1 % à 25 % en poids par rapport à la composition totale d'agent de protection de la peau.

**3.** Utilisation selon les revendicationss 1 à 2,
**caractérisée en ce que**
le mélange émulsifiant est utilisé dans des quantités de 5 % à 15 % en poids par rapport à la consommation totale d'agent de protection de la peau.

**4.** Utilisation selon les revendicationss 1 à 3,
**caractérisée en ce que**
le mélange émulsifiant contient au moins un poly-12-hydroxystéarate de polyol en tant que composant émulsifiant.

**5.** Utilisation selon les revendicationss 1 à 4,
**caractérisée en ce que**
le mélange émulsifiant contient en tant que poly-12-hydroxystéarates de polyol des poly-12-hydroxystéarates de polyglycérol en tant que composant émulsifiant.

**6.** Utilisation selon les revendicationss 1 à 5,
**caractérisée en ce qu'**
on utilise des PEG-30-dipolyhydroxystéarates en tant que dipolyhydroxystéarates éthoxylés.

**7.** Utilisation selon les revendicationss 1 à 6,
**caractérisée en ce que**
la fraction d'huiles apolaires ne dépasse pas 15 % en poids dans l'agent de protection de la peau.

**8.** Utilisation selon les revendicationss 1 à 7,
**caractérisée en ce que**
la fraction d'huiles apolaires ne dépasse pas 10 % en poids dans l'agent de protection de la peau.

**9.** Utilisation selon les revendicationss 1 à 8,
**caractérisée en ce que**
l'agent de protection de la peau contient du bisabolol dans une quantité de 0,1 % à 5 % en poids.

**10.** Utilisation selon les revendicationss 1 à 9,
**caractérisée en ce que**
l'agent de protection de la peau contient du bisabolol dans une quantité de 0,5 % à 1,5 % en poids.

**11.** Utilisation selon les revendicationss 1 à 10,
**caractérisée en ce que**
l'agent de protection de la peau contient des tanins végétaux naturels dans une quantité de 0,1 % à 2 % en poids.

**12.** Utilisation selon les revendicationss 1 à 11,
**caractérisée en ce que**
l'agent de protection de la peau contient des tanins végétaux naturels dans une quantité de 0,4 % à 0,8 % en poids.

**13.** Agent de protection de la peau sans silicones destiné à la protection contre les NOx aqueux, pouvant être obtenu à partir d'émulsions E/H/E préparées avec un mélange émulsifiant constitué de poly-12-hydroxystéarates de polyol en combinaison avec un glucoside d'alkyle et/ou d'alkylène et d'un alcool gras et/ou de glycérides partiels, le mélange émulsifiant contenant en outre au moins un dipolyhydroxystéarate éthoxylé et la fraction d'huiles apolaires dans l'agent de protection de la peau ne dépasse pas 20 % en poids.

**14.** Agent de protection de la peau selon la revendication 13,
**caractérisé en ce qu'**
on utilise le mélange émulsifiant dans des quantités de 1 % à 25 % en poids par rapport à la préparation totale d'agent de protection de la peau.

**15.** Agent de protection selon les revendications 13 à 14,
**caractérisé en ce qu'**
on utilise le mélange émulsifiant dans des quantités de 5 % à 15 % en poids par rapport à la composition totale d'agent de protection de la peau.

**16.** Agent de protection selon les revendications 13 à 15,
**caractérisé en ce que**
le mélange émulsifiant contient au moins un poly-12-hydroxystéarate de polyol en tant que composant émulsifiant.

**17.** Agent de protection de la peau selon les revendications 13 à 16,

**caractérisé en ce que**

le mélange émulsifiant contient en tant que poly-12-hydroxystéarates de polyol des poly-12-hydroxystéarates de polyglycérol en tant que composant émulsifiant.

**18.** Agent de protection de la peau selon les revendications 13 à 17,
**caractérisé en ce qu'**
on utilise comme dipolyhydroxystéarates éthoxylés des PEG-30-dipolyhydroxystéarates.

**19.** Agent de protection de la peau selon les revendications 13 à 18,
**caractérisé en ce que**
la fraction d'huiles apolaires ne dépasse pas 15 % en poids dans celui-ci.

**20.** Agent de protection de la peau selon les revendications 13 à 19,
**caractérisé en ce que**
la fraction d'huiles apolaires ne dépasse pas 10 % en poids dans l'agent de protection de la peau.

**21.** Agent de protection de la peau selon les revendications 13 à 20,
**caractérisé en ce qu'**
il contient du bisabolol dans une quantité de 0,1 % à 5 % en poids.

**22.** Agent de protection de la peau selon les revendications 13 à 21,
**caractérisé en ce qu'**
il contient des tanins végétaux naturels dans une quantité de 0,1 % à 2 % en poids.

**23.** Agent de protection de la peau selon les revendications 13 à 22,
**caractérisé en ce qu'**
il contient des tanins végétaux naturels dans une quantité de 0,4 % à 0,8 % en poids.

## Claims

**1.** Use of multiple W/O/W emulsions for the preparation of silicon-free skin protecting agents for protection from aqueous noxious substances, **characterised in that** the W/O/W emulsions are formed using an emulsifier mixture comprising polyolpoly-12-hydroxystearates combined with an alkyl and/or alkylene glucoside and a fatty alcohol and/or partial glycerides, the emulsifier mixture additionally containing at least one ethoxylated dipolyhydroxystearate and the proportion of non-polar oils in the skin protecting agent not exceeding 20 wt.%.

**2.** Use according to claim 1, **characterised in that** the emulsifier mixture is used in amounts of from 1 to 25 wt.%, based on the total composition of the skin protecting agent.

**3.** Use according to claims 1 to 2, **characterised in that** the emulsifier mixture is used in amounts of from 5 to 15 wt.%, based on the total composition of the skin protecting agent.

**4.** Use according to claims 1 to 3, **characterised in that** the emulsifier mixture contains at least one polyolpoly-12-hydroxystearate as an emulsifier component.

**5.** Use according to claim 1 to 4, **characterised in that** the emulsifier mixture contains, as polyolpoly-12-hydroxystearates, polyglycerolpoly-12-hydroxystearates as emulsifier component.

**6.** Use according to claims 1 to 5, **characterised in that** PEG-30 dipolyhydroxystearates are used as ethoxylated dipolyhydroxystearates.

**7.** Use according to claims 1 to 6, **characterised in that** the proportion of non-polar oils in the skin protecting agent does not exceed 15 wt.%.

**8.** Use according to claims 1 to 7, **characterised in that** the proportion of non-polar oils in the skin protecting agent does not exceed 10 wt.%.

9.  Use according to claims 1 to 8, **characterised in that** bisabolol is present in the skin protecting agent in an amount of from 0.1 to 5 wt.%.

10. Use according to claims 1 to 9, **characterised in that** bisabolol is present in the skin protecting agent in an amount of from 0.5 to 1.5 wt.%.

11. Use according to claims 1 to 10, **characterised in that** natural vegetable tannins are present in the skin protecting agent in an amount of from 0.1 to 2 wt.%.

12. Use according to claims 1 to 11, **characterised in that** natural vegetable tannins are present in the skin protecting agent in an amount of from 0.4 to 0.8 wt.%.

13. Silicon-free skin protecting agents for protection from aqueous noxious substances, obtainable from W/O/W emulsions which are formed using an emulsifier mixture comprising polyolpoly-12-hydroxystearates combined with an alkyl and/or alkylene glucoside and a fatty alcohol and/or partial glycerides, the emulsifier mixture additionally containing at least one ethoxylated dipolyhydroxystearate and the proportion of non-polar oils not exceeding 20 wt.% in the skin protecting agent.

14. Skin protecting agent according to claim 13, **characterised in that** the emulsifier mixture is used in amounts of from 1 to 25 wt.%, based on the total composition of the skin protecting agent.

15. Skin protecting agent according to claims 13 to 14, **characterised in that** the emulsifier mixture is used in amounts of from 5 to 15 wt.%, based on the total composition of the skin protecting agent.

16. Skin protecting agent according to claims 13 to 15, **characterised in that** the emulsifier mixture contains at least one polyolpoly-12-hydroxystearate as emulsifier component.

17. Skin protecting agent according to claims 13 to 16, **characterised in that** the emulsifier mixture contains, as polyolpoly-12-hydroxystearates, polyglycerolpoly-12-hydroxystearates as emulsifier component.

18. Skin protecting agent according to claims 13 to 17, **characterised in that** PEG-30 dipolyhydroxystearates are used as ethoxylated dipolyhydroxystearates.

19. Skin protecting agent according to claims 13 to 18, **characterised in that** the proportion of non-polar oils in the skin protecting agent does not exceed 15 wt.%.

20. Skin protecting agent according to claims 13 to 19, **characterised in that** the proportion of non-polar oils in the skin protecting agent does not exceed 10 wt.%.

21. Skin protecting agent according to claims 13 to 20, **characterised in that** bisabolol is present in the skin protecting agent in an amount of from 0.1 to 5 wt.%.

22. Skin protecting agent according to claims 13 to 21, **characterised in that** natural vegetable tannins are present in the skin protecting agent in an amount of from 0.1 to 2 wt.%.

23. Skin protecting agent according to claims 13 to 22, **characterised in that** natural vegetable tannins are present in the skin protecting agent in an amount of from 0.4 to 0.8 wt.%.

# DIAGRAMM I

## Repetitiver okklusiver Irritationstest:
## Barriere-Schaden (TEWL-Anstieg)

schlechter

prozentuale
Barriereschädigung

besser

100%

80%

60%

48%

40%

20%

0%

64 %

71%

100%

(W/O/W)  Vaseline DAB  Schutzprodukt  0,5 % NaLS ohne
                        vom Typ W/O   Hautschutz

11

# DIAGRAMM II

## Regenerationswirkung
## (Barrier-Recovery; TEWL)